# EUROPÄISCHE PATENTSCHRIFT

(11) **EP 2 547 372 B1**
(45) Veröffentlichungstag und Bekanntmachung des Hinweises auf die Patenterteilung: **10.05.2017**
(21) Anmeldenummer: 11718257.6
(22) Anmeldetag: 15.03.2011
(51) Int. Cl.: A61L 27/48, C12P 19/04

(54) **MEHRPHASIGE BIOMATERIALIEN AUF BASIS BAKTERIELL SYNTHETISIERTER NANOCELLULOSE UND VERFAHREN ZU IHRER HERSTELLUNG**
MULTI-PHASE BACTERIALLY-SYNTHESIZED-NANOCELLULOSE BIOMATERIALS AND METHOD FOR PRODUCING SAME
BIOMATÉRIAUX MULTIPHASÉS À BASE DE NANOCELLULOSE SYNTHÉTISÉE PAR DES BACTÉRIES (BNC) ET LEUR PROCÉDÉ DE FABRICATION

(30) Priorität: 19.03.2010 DE 102010012437
(43) Veröffentlichungstag der Anmeldung: 23.01.2013
(73) Patentinhaber: JeNaCell GmbH, 07745 Jena (DE)
(72) Erfinder: HESSLER, Nadine, 96529 Mengersgereuth-Hämmern (DE); SULTANOVA, Barno, 10025 Tashkent (UZ); KLEMM, Dieter, 99425 Weimar (DE)
(74) Vertreter: Fuchs Patentanwälte Partnerschaft mbB
(86) Internationale Anmeldenummer: PCT/DE2011/000269
(87) Internationale Veröffentlichungsnummer: WO 2011/113423

(56) Entgegenhaltungen:
- US-A1- 2007 053 960
- US-A1- 2009 209 897

## Beschreibung

Die Erfindung betrifft mehrphasige Biomaterialien auf Basis bakteriell synthetisierter Nanocellulose und ein Verfahren zu ihrer Herstellung. Die vorgeschlagenen BNC-Materialien zeichnen sich für breite Anwendung beispielsweise in der Medizin (Wundauflagen, unterschiedlichste Implantate), in der Technik (Membrane, Folien, Barriereschichten) und in der Lebensmittelbranche (kalorienfreie Ernährung, Verpackung) durch weitgehend universell bestimmbare Strukturen und Materialeigenschaften aus.
Dieses anwendungsspezifische Erzielen definierter und sogar für BNC-Materialien an sich neuer Strukturen und Eigenschaften betrifft insbesondere die mechanischen Festigkeiten, Elastizität, Transparenz und des Wasserhaushaltes, speziell die Fähigkeit zu einer angemessenen bis vollständigen Requellung nach der Trocknung, wie auch so genannte Filter-/Membran-Funktionen (Permeabilität) und Scaffold-Eigenschaften (Porensystem, Oberflächencharakteristik, Besiedelung durch Zellen) sowie die Biokompatibilität (Körperverträglichkeit, Endothelisierung, Einwanderung körpereigener Zellen, dauerhafte Integration in den Körper), ohne dass nachteilige Additive bzw. bei der Synthese mit diesen entstehenden Kompositbildungen erforderlich werden.

Es ist allgemein bekannt, zur Beeinflussung von homogenen oder mehrphasigen Biomaterialien auf Basis von bakteriell synthetisierter Nanocellulose (BNC) dieses Material nach dessen Synthese (*post* Modifizierung) zu verändern (K.-Y. Lee, J.J. Blaker, A. Bismarck: Surface functionalisation of bacterial cellulose as the route to produce green polylactide nanocomposites with improved properties, Composites Science and Technology (2009), 69(15-16), 2724-2733; D. Klemm, D. Schumann, F. Kramer, N. Heßler, M. Hornung, H.-P. Schmauder, S. Marsch: Nanocelluloses as Innovative Polymers in Research and Application. Advances in Polymer Science (2006), 205(Polysaccharides II), 49-96).
Möglich ist hingegen auch, bereits mit der Synthese des biotechnologischen Kultivierungsprozesses eine *in situ* Modifizierung vorzunehmen (H. Wang, F. Guan, X. Ma, S. Ren: Production and performance determination of modified bacterial cellulose, Shipin Keji (2009), (5), 28-31; N. Hessler, D. Klemm: Alteration of bacterial nanocellulose structure by in situ modification using polyethylene glycol and carbohydrate additives, Cellulose (Dordrecht, Netherlands) (2009), 16(5), 899-910; D. Klemm, D. Schumann, F. Kramer, N. Heßler, M. Hornung, H.-P. Schmauder, S. Marsch: Nanocelluloses as Innovative Polymers in Research and Application. Advances in Polymer Science (2006), 205(Polysaccharides II), 49-96).
In diesem Fall werden während der Biosynthese unterschiedliche Additive zum Nährmedium zugegeben (beispielsweise M. Seifert: Modifizierung der Struktur von Bakteriencellulose durch die Zusammenstellung des Nährmediums bei der Kultivierung von *Acetobacter xylinum*, Promotionsarbeit, Friedrich-Schiller-Universität Jena, 2004; O.M. Astley, E. Chanliaud, A.M. Donald, M.J. Gidley: Structure of Acetobacter cellulose composites in the hydrated state, International journal of biological macromolecules (2001), 29/3, 193-202; N. Sakairi, H. Asano, M. Ogawa, N. Nishi, S. Tokura: A method for direct harvest of bacterial cellulose filaments during continuous cultivation of acetobacter xylinum. Carbohydrate Polymers (1998), 35/3-4, 233-7; C.H. Haigler, A.R. White, R.M. Brown Jr., K.M. Cooper: Alteration of In Vivo Cellulose Ribbon Assembly by Carboxymethylcellulose and Other Cellulose Derivative, J Cell Biology (1982), 94, 64-9).
Demnach hat der Zusatz von Carboxymethylcellulose (CMC) sowie Methylcellulose (MC) enorme Auswirkungen auf das BNC-Netzwerk. Beide Zusätze nehmen infolge ihrer Einlagerung Einfluss auf das Porensystem und die daraus resultierenden Eigenschaften, wie z. B. Elastizität, Wasserauf- bzw. Wasserrückhaltevermögen, Filterfunktion, wodurch neuartige BNC-Materialien entstehen (O.M. Astley, E. Chanliaud, A.M. Donald, M.J. Gidley: Structure of Acetobacter cellulose composites in the hydrated state, International journal of biological macromolecules (2001), 29/3, 193-202; N. Sakairi, H. Asano, M. Ogawa, N. Nishi, S. Tokura: A method for direct harvest of bacterial cellulose filaments during continuous cultivation of acetobacter xylinum. Carbohydrate Polymers (1998), 35/3-4, 233-7; C.H. Haigler, A.R. White, R.M. Brown Jr., K.M. Cooper: Alteration of In Vivo Cellulose Ribbon Assembly by Carboxymethylcellulose and Other Cellulose Derivative, J Cell Biology (1982), 94, 64-9).

Des Weiteren war der Zusatz von pflanzlichen Zellwand-Begleitkomponenten, wie Xyloglucan oder Pektin, zum Nährmedium während der BNC-Biosynthese Bestandteil von Untersuchungen, um strukturelle Zusammenhänge von nativer Cellulose zu imitieren und deren Bildung im Detail zu analysieren (J. Cybulska, E. Vanstreels, Q.T. Ho, C.M. Courtin, V. Van Craeyveld, B. Nicolai, A. Zdunek, K. Konstankiewicz: Mechanical characteristics of artificial cell walls, Journal of Food Engineering (2009), 96(2), 287-294).

Im Gegensatz zu wasserlöslichen Verbindungen lassen sich auch während der Biosynthese Feststoffe als Additive zum Nährmedium zugeben, die in das gebildete BNC-Netzwerk eingebaut werden. Während Udhardt (U. Udhardt: Synthese, Eigenschaften und Strukturdesign von Bakteriencellulose mit speziellem Anwendungspotential von BASYC^{®}-Implantaten in der Mikrochirurgie, Promotionsarbeit, Friedrich-Schiller-Universität Jena, 2004) eine Einlagerung von Glaskugeln bzw. einen Einbau von Kieselgel sowie anorganischen Salzen (Calciumcarbonat) in das BNC-Netzwerk beschrieb, schilderten Serafica et al. (G. Serafica, R. Mormino, H. Bungay: Inclusion of solid particles in bacterial cellulose, Applied Microbiology and Biotechnology (2002), 58/6, 756-60) vor allem die Integration von Metallen (Aluminium) bzw. Metalloxiden (Eisenoxid)-Partikeln.

Der Nachteil dieser *in situ* Methoden liegt jedoch in der Notwendigkeit von Additiven, um neuartige Biomaterialien auf BNC-Basis herzustellen. So ist nur mit Hilfe von wasserlöslichen organischen, anorganischen Substanzen oder Polymeren sowie Feststoffpartikeln eine Steuerung der Struktur und den damit verbundenen Eigenschaften möglich. Des Weiteren bringen die eingelagerten Additive in der Verwendung als Medizinprodukt das Risiko von möglichen allergischen Reaktionen gegenüber reiner BNC mit sich.
Im Falle der *post* Modifizierung gelingt eine Modifizierung der BNC sowie die Erzeugung homogener oder mehrphasiger Materialien durch das Einbringen von organischen bzw. anorganischen Substanzen nach der Kultivierung (B.R. Evans, H. O'Neil, M. Hugh, V.P. Malyvanh, I. Lee, J. Woodward: Palladium-bacterial cellulose membranes for fuel cells, Biosensors & Bioelectronics (2003), 18/7, 917-23; B.R. Evans, H.M. O'Neill, E. Greenbaum: Electron Transfer by Enzymes and Photosynthetic Proteins Immobilized in Polysaccharide Composites, Abstracts, 57th Southeast/61st Southwest Joint Regional Meeting of the American Chemical Society, Memphis, TN, United States, November 1-4, 2005; W.A. Daoud, J.H. Xin, Y.-H.Zhang: Surface functionalization of cellulose fibers with titanium dioxide nanoparticles and their combined bactericidal activities, Surface Science (2005), 599(1-3), 69-75; D. Zhang, L. Qi: Synthesis of mesoporous titania networks consisting of anatase nanowires by templating of bacterial cellulose membranes, Chem. Commun. (2005), 21, 2735-7).
Mit Hilfe dieser Methode wurden bereits eine Vielzahl von BNC-Variationen, z. B. durch die Benutzung verschiedener Typen von Monomeren und synthetischen Polymeren (H. Yano, S. Nakahara: Biocomposites produced from plant microfiber bundles with a nanometer unit web-like network, Journal of Materials Science (2004), 39/5, 1635-8; V. Dubey, L.K. Pandey, C. Saxena: Pervaporative separation of ethanol/water azeotrope using a novel chitosan-impregnated bacterial cellulose membrane and chitosan-poly(vinyl alcohol) blends, Journal of Membrane Science (2005), 251(1-2), 131-136; V. Dubey, C. Saxena, L. Singh, K.V. Ramana, R.S. Chauhan: Pervaporation of binary waterethanol mixtures through bacterial cellulose membrane, Separation and Purification Technology (2002), 27/2, 163-71; W.A. Daoud, J.H. Xin, Y.-H. Zhang: Surface functionalization of cellulose fibers with titanium dioxide nanoparticles and their combined bactericidal activities, Surface Science (2005), 599(1-3), 69-75), strukturbildenden Polymeren, wie PVA (T. Wan, Y. Zhu: Preparation of bacterial cellulose/poly(vinyl alcohol) composite gels, Faming Zhuanli Shenqing Gongkai Shuomingshu CN 101570616, 2009), Gelatine (K. Yasuda, J.P. Gong, Y. Katsuyama, A. Nakayama, Y. Tanabe, E. Kondo, M. Ueno, Y. Osada: Biomechanical properties of high-toughness double network hydrogels, Biomaterials (2005), 26/2, 4468-75; A. Nakayama, A. Kakugo, J.P. Gong, Y. Osada, M. Takai, T. Erata, S. Kawano: High mechanical strength double-network hydrogel with bacterial cellulose, Advanced Functional Materials (2004), 14/11, 1124-8) sowie anorganischen Substanzen, wie Calciumsalzen, Metallen, Metalloxiden, (B.R. Evans, H. O'Neil, M. Hugh, V.P. Malyvanh, I. Lee, J. Woodward: Palladium-bacterial cellulose membranes for fuel cells, Biosensors & Bioelectronics (2003), 18/7, 917-23; B.R. Evans, H.M. O'Neill, E. Greenbaum: Electron Transfer by Enzymes and Photosynthetic Proteins Immobilized in Polysaccharide Composites, Abstracts, 57th Southeast/61st Southwest Joint Regional Meeting of the American Chemical Society, Memphis, TN, United States, November 1-4, 2005; Daoud, J.H. Xin, Y.-H.Zhang: Surface functionalization of cellulose fibers with titanium dioxide nanoparticles and their combined bactericidal activities, Surface Science (2005), 599(1-3), 69-75; D. Zhang, L. Qi: Synthesis of mesoporous titania networks consisting of anatase nanowires by templating of bacterial cellulose membranes, Chem. Commun. (2005), 21, 2735-7) realisiert.

Jedoch besteht der Nachteil hier zum einen darin, dass zwei Herstellungsstufen (Synthese der BNC sowie deren Modifizierung) zur Entwicklung neuwertiger BNC notwendig sind. Außerdem verändert die *post* Modifizierung die BNC teilweise in solchem Maß, dass die einzigartige Struktur und damit die herausragenden Eigenschaften verloren gehen. Andererseits liegt auch in diesen Fällen der nachteilige Gebrauch von Additiven vor.

Eine andere Lösung zur Darstellung von neuen BNC-Materialien beinhaltet die gemeinsame Kultivierung von Bakterien unterschiedlicher Stämme. So zeigten A. Seto et al. (A. Seto, Y. Saito, M. Matsushige, H. Kobayashi, Y. Sasaki, N. Tonouchi, T. Tsuchida, F. Yoshinaga, K. Ueda, T. Beppu: Effective cellulose production by a coculture of Gluconacetobacter xylinus and Lactobacillus mali, Applied Microbiology and Biotechnology (2006), 73(4), 915-921), C. Choi et al. (KR 2002/067226) sowie H. Seto et al. (JP 10201495), dass durch die Co-Kultivierung eines cellulosebildenden Bakterienstammes (*Acetobacter xylinum* (st-60-12)) mit einem Lactobacillus-Stamm (*Lactobacillus mali* (st-20)) die Ausbeute der synthetisierten Cellulose optimiert werden konnte. Dies basiert vor allem auf den Stoffwechselprodukten, wie z. B. Essigsäure, des Lactobacillus-Stammes, welche die Biosynthese der Cellulose unterstützen (A. Seto, Y. Saito, M. Matsushige, H. Kobayashi, Y. Sasaki, N. Tonouchi, T. Tsuchida, F. Yoshinaga, K. Ueda, T. Beppu: Effective cellulose production by a coculture of Gluconacetobacter xylinus and Lactobacillus mali, Applied Microbiology and Biotechnology (2006), 73(4), 915-921; KR 2002/067226; JP 10201495). Im Gegensatz dazu führte die Co-Kultivierung von *Acetobacter aceti* subsp. *xylinum* (NCI 1005) mit den Stämmen ATCC 10245 oder NCI 1051 zur Steigerung der jeweiligen Polymer-Synthese. So bewirken einerseits die zusätzliche Cellulose-Produktion und deren anschließender Abbau eine Zunahme der Nährstoffe in der Kulturlösung, wodurch die Ausbeute der Polymere erhöht wurde. Andererseits ermöglichte die Gegenwart von Cellulose in der Kulturlösung die Bildung von wasserlöslichen verzweigten Polysacchariden (K. Tajima, H. Ito, M. Fujiwara, M. Takai, J. Hayashi: Enhancement of bacterial cellulose productivity and preparation of branched polysaccharide-bacterial cellulose composite by co-cultivation of Acetobacter species, Sen'i Gakkaishi (1995), 51(7), 323-32; K. Tajima, M. Fujiwara, M. Takai: Biological control of cellulose. Macromolecular Symposia (1995), 99 (Functional Polysaccharides), 149-55). US2007/0053960 beschreibt ein biokompatibeles Implantat oder eine Wundauflage, bestehend aus mikrobieller Cellulose.

Der Fachwelt sind jedoch ausschließlich Co-Kultivierungsmethoden bekannt, welche sich auf die Steigerung der Produktivität der Cellulose-Gewinnung oder auf eine Kompositbildung beziehen, wobei jeweils ein zur Cellulosesynthese bekannter celluloseproduzierender Bakterienstamm kultiviert wird.

Eine Kultivierung mehrerer unterschiedlicher Bakterienstämme zum Zweck der Beeinflussung von Struktur und Eigenschaften der BNC ist hingegen nicht bekannt geworden.
Modifizierungen der BNC-Eigenschaften werden ausschließlich durch Additive bewirkt, die zur Kultivierung oder nach derselben hinzugegeben werden und sich in die BNC-Struktur einlagern. Außerdem ist die Zugänglichkeit von mehrphasigen Biomaterialsystemen stark eingeschränkt, da durch eine resultierende Kompositbildung lediglich homogene Strukturen erreichbar sind.

Der Erfindung liegt die Aufgabe zu Grunde, ohne erforderliche Additive und Kompositbildungen mehrphasige Biomaterialien auf Basis bakteriell synthetisierter Nanocellulose zu schaffen, deren Bakteriencellulose-Eigenschaften bei der Synthese in sehr weiten Grenzen definiert beeinflusst werden können.

Erfindungsgemäß werden die Biomaterialien auf Basis bakteriell synthetisierter Nanocellulose aus mindestens zwei verschiedenen Cellulose-bildenden Bakterienstämmen zu mehreren, das heißt zu wenigstens zwei, unterschiedlichen Bakteriencellulose-Netzwerken in einem gemeinsamen Kulturmedium miteinander synthetisiert. Damit werden die Eigenschaften der Bakteriencellulose nicht durch extra zugegebene Additive oder bei der Synthese mit diesen entstehenden Kompositbildungen erzielt, sondern durch gesteuerte Generierung des synthetisierten Phasensystems, bestehend aus den mehreren unterschiedlichen Bakteriencellulose-Netzwerken.

Die besagten Bakteriencellulose-Netzwerke, die sich insbesondere in ihrer molekularen und/oder supramolekularen Struktur unterscheiden, können beispielsweise als kombiniertes homogenes Phasensystem synthetisiert werden und somit eine gemeinsame homogene Phase des Biomaterials ausbilden.

Auch eine verknüpfte Ausbildung der Phasensysteme kann generiert werden, indem die zumindest zwei unterschiedlichen Bakteriencellulose-Netzwerke als schichtförmiges Phasensystem, bestehend aus wenigstens einer kombinierten homogenen Phase sowie aus wenigstens einer Einzelphase, ausgebildet sind.

Je nach Auswahl und Anzahl der verwendeten, unterschiedlichen Cellulose-bildenden Bakterienstämme für die Synthese sowie der Wahl der Synthesebedingungen, insbesondere durch die Zusammensetzung des Kulturmediums, werden allein durch die synthetisierten Bakteriencellulose-Netzwerke, und damit ohne nachteilige zwingend erforderliche Zusätze als Ausgangskomponenten der Synthese neue Biomaterialien generiert, deren Bakteriencellulose-Eigenschaften im besagten Syntheseprozess in sehr weiten Grenzen beeinflusst und damit bei der Herstellung definiert gesteuert werden können.

Diese Bakteriencellulose-Eigenschaften betreffen beispielsweise die gezielte Vergrößerung oder Verkleinerung der Porengröße sowie die Transparenz und Stabilität der BNC-Materialien, wie sie durch die an sich bekannte Kultivierung bzw. durch die Verwendung von *in situ* oder *post* Modifizierung ausschließlich eines der bekannten Bakterienstämme nicht erhalten werden konnte.

Selbst das Erreichen für die Synthese von BNC-Materialien an sich widersprüchlicher Eigenschaften, wie sehr hoher Wassergehalt bei gallertiger, weicher Konsistenz und dichte Materialstruktur hoher Festigkeit, in ein und demselben Material bakteriell synthetisierter Nanocellulose sind überraschend realisierbar und könnten hier neue Einsatzmöglichkeiten für BNC-Materialien erschließen.

Die Struktur und die Eigenschaften der BNC-Materialien können durch volumetrische Verhältnis der wässrigen Zelldispersionen der verwendeten Bakterienstämme in sehr weiten Grenzen definiert bestimmt und bei der Synthese "maßgeschneidert" kontrolliert werden. Dieses "Maßschneidern" ist auf alle Strukturen und Eigenschaften anwendbar, welche für die Applikation von BNC-Materialien in feuchter oder getrockneter (heißgepresster, luft- oder gefriertrockner) Form beispielsweise in der Medizin (Wundauflagen, Implantate), in der Technik (Membrane, Folien, Barriereschichten) und in der Nahrungsmittelindustie (kalorienfreie Ernährung, Verpackung) relevant sind. Das betrifft die Steuerung der mechanischen Festigkeit, Elastizität, Permeabilität, Transparenz und des Wasserhaushaltes ebenso wie so genannte Scaffold-Eigenschaften (Porensystem, Oberfächencharakteristik, Besiedelung durch Zellen) und auch die Biokompatibilität (Körperverträglichkeit, Endothelisierung, Einwanderung körpereigener Zellen, dauerhafte Integration in den Körper).

Im Syntheseprozess können Struktur und Eigenschaften der BNC-Materialien insbesondere durch die Variation der Kultivierung (Zusammenführen der Bakterienstämme vor oder während der Beimpfung) der jeweiligen Cellulose-produzierenden Bakterienstämme, der Gebrauch von verschiedenen Kultivierungsmedien oder durch die Verwendung von unterschiedlichen Kultivierungsparametern (Temperatur, Dauer, Volumen, Kultivierungsgefäße) beeinflusst werden.

Die Erfindung ist nicht auf so genannte "reine" BNC-Materialien beschränkt, sondern schließt auch die Verwendung von speziellen Bakterienstämmen ein, die celluloseartige Strukturen auf Basis von modifizierten C-Quellen produzieren, wie beispielsweise bei der Verwendung von N-Acetylglucosamin oder Glucosamin als C-Quelle.

Die Erfindung soll nachstehend anhand von in der Zeichnung dargestellten Ausführungsbeispielen näher erläutert werden.

Es zeigen:
- Fig. 1:: Bakteriell synthetisierte Nanocellulose (BNC), bestehend aus mehreren unterschiedlichen Bakteriencellulose-Netzwerken, die ein gemeinsames homogenes Phasensystem bilden
- Fig. 2:: BNC mit zwei unterschiedlichen Bakteriencellulose-Netzwerken, die ein schichtförmiges Phasensystem, bestehend aus zwei schichtförmigen Einzelphasen sowie einer kombinierten homogenen Phase, bilden

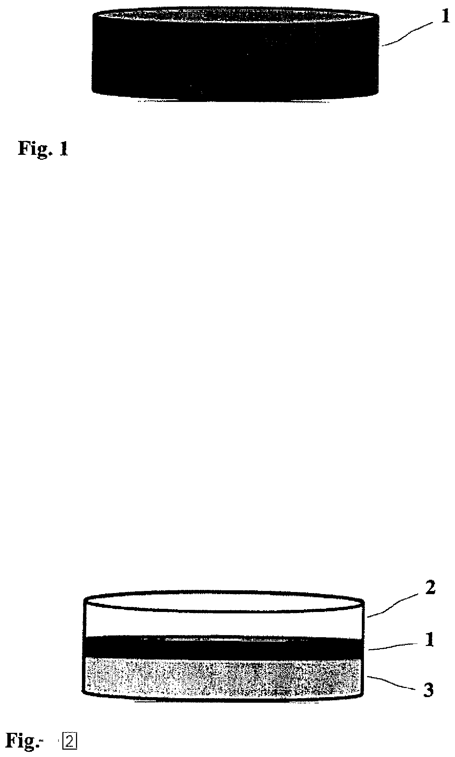

In Fig. 1 ist eine bakteriell synthetisierte Nanocellulose (BNC-Biomaterial) dargestellt, die erfindungsgemäß aus mehreren (im Beispiel zwei) unterschiedlichen Bakteriencellulose-Netzwerken besteht, welche ein gemeinsames Phasensystem aus einer kombinierten homogenen Phase (1) bilden.
Synthetisiert wird dieses Phasensystem aus zwei Arten von *Gluconacetobacter* Stämmen, in diesem Beispiel ATCC 23769 und DSM 11804, in einem nicht dargestellten Kulturgefäß mit einer Synthesefläche von 7 cm², welche jedoch für die spezielle Phasenbildung in diesem Ausführungsbeispiel frei wählbar ist. Die beiden Bakterienstämme werden nach separater Präparation zusammen in das Kulturgefäß gegeben und somit für die gemeinsame Synthese beimpft.
Ein zugegebenes Kultivierungsmedium, besteht aus einer Kohlenstoffquelle (vorzugsweise verschiedene Zucker und deren Derivate), aus einer Stickstoff-Quelle (vorzugsweise Pepton), aus einer Vitaminquelle (vorzugsweise Hefeextrakt) sowie gegebenenfalls aus einem Puffersystem (vorzugsweise Dinatriumhydrogenphosphat und Zitronensäure).
Die Biosynthese wurde bei 28-30°C für 3-21 Tage durchgeführt und sowohl für ein diskontinuierliches als auch für ein kontinuierliches Syntheseverfahren erprobt.
Mit einem Verhältnis 5 : 1 bzw. 2 : 1 zwischen Nährmedium und den Bakterienstämmen wird ein gemeinsames, sehr stabiles und transparentes kombiniertes homogenes BNC-Phasensystem (siehe Fig. 1) aus beiden synthetisierten BNC-Netzwerken erzielt.
Das so genannte Beimpfungsverhältnis (Verhältnis der beimpften Bakterienstämme zueinander) ist 50 : 50 (ATCC 23769 : DSM 11804), d. h. die Bakterienstämme gehen zu gleichen Mengenanteilen in die Synthese. Eine Veränderung dieses Beimpfungsverhältnisses würde zusätzlich eine Steuerung des Porensystems und somit der Stabilität sowie eine Beeinflussung der Transparenz des homogenen BNC-Biomaterials ermöglichen. Bei einem Beimpfungsverhältnis von 10 : 90 zeigte sich beispielsweise die Bildung eines festen/stabilen, durchsichtigen und gleichzeitig elastischen BNC-Vlieses. Durch gegenläufige Veränderung dieses Beimpfungsverhältnisses (beispielsweise 90 : 10) können sowohl Festigkeit als auch Elastizität unter Erhalt der Transparenz verringert werden.

Außerdem kann durch das Hinzufügen von Eisessig bis zu 2 % zusätzlich die Homogenität des entstehenden BNC-Biomaterials verbessert werden.

In Fig 2 ist ein BNC-Biomaterial dargestellt, das vorschlagsgemäß ebenfalls aus zwei unterschiedlichen Bakteriencellulose-Netzwerken besteht, welche jedoch zu einem speziellen schichtförmigen Phasensystem synthetisiert wurde, wobei zwei separate Einzelphasen (2, 3) jeweils einem jeweiligen BNC-Vlies des korrespondieren Bakterienstammes und dessen an sich bekannten Eigenschaften entsprechen und beide Einzelphasen (2,3) über eine kombinierte homogene Phase (1) fest miteinander verbunden sind.

Synthetisiert wird dieses spezielle Phasensystem aus den zwei *Gluconacetobacter* Stämmen ATCC 23769 und DSM 14666 wiederum in dem besagten nicht dargestellten Kulturgefäß mit einer Synthesefläche von 7 cm². Durch Veränderung dieser Synthesefläche kann bei dieser Kultivierung ein gezielter Einfluss auf die Bildung der Einzelphasen 2, 3 genommen werden, wobei mit Flächenvergrößerung (Beimpfungsverhältnis von 50 : 50) die Ausbildung der Einzelphase 2 (korrespondierend zum Bakterienstamm DSM 14666) gegenüber Einzelphase 3 (korrespondierend zum Bakterienstamm ATCC 23769) bevorzugt wird.
Das in Fig. 2 dargestellte Phasensystem des BNC-Biomaterials wird durch die Verwendung der genannten Bakterienstämme sowie durch deren separate Präparation und nachfolgende gemeinsame Beimpfung erreicht. Eine gemeinsame Kultivierung dieser Bakterienstämme, einschließlich gemeinsamer Präparation, würde hingegen ein kombiniertes homogenes Phasensystem (vgl. Fig. 1) ausbilden.

Als Kulturmedium kommt auch hier eine Mischung aus einer Kohlenstoffquelle (vorzugsweise verschiedene Zucker und deren Derivate), aus einer Stickstoff-Quelle (vorzugsweise Pepton), aus einer Vitaminquelle (vorzugsweise Hefeextrakt) sowie gegebenenfalls aus einem Puffersystem (vorzugsweise Dinatriumhydrogenphosphat und Zitronensäure) zum Einsatz.
Die Biosynthese wurde bei 28-30°C für 3-21 Tage mit einem Verhältnis von 20: 1 zwischen Nährmedium und den Bakterienstämmen durchgeführt und sowohl für ein diskontinuierliches als auch für ein kontinuierliches Syntheseverfahren erprobt.
Das Beimpfungsverhältnis von 50 : 50 zwischen den Bakterienstämmen führt zu dem äußerlich sichtbaren schichtförmigen BNC-Phasensystem (Fig. 3) mit den besagten zwei Einzelphasen 2, 3 und der dazwischen liegenden homogene Phase 1. Überdies liegen die Einzelphasen bei diesem Beimpfungsverhältnis zu gleichen Teilen vor.
Je nach Verschiebung des Beimpfungsverhältnisses zu Gunsten eines Bakterienstammes kann jeweils die Dicke der Einzelphasen 2, 3 sowie die resultierenden Eigenschaften (Wasseraufnahme- und Wasserrückhaltevermögen usw.) gezielt beeinflusst werden.

Aufstellung der verwendeten Bezugszeichen
- 1: - kombinierte homogene Phase
- 2,3: - separate Einzelphase

## Patentansprüche

1. Mehrphasige Biomaterialien auf Basis bakteriell synthetisierter Nanocellulose (BNC),bestehend aus zumindest zwei unterschiedlichen Bakteriencellulose-Netzwerken, **dadurch gekennzeichnet, dass** die zumindest zwei unterschiedlichen Bakteriencellulose-Netzwerke als kombiniertes homogenes Phasensystem (1) oder als schichtförmiges Phasensystem, bestehend aus wenigstens einer kombinierten homogenen Phase (1) sowie aus wenigstens einer Einzelphase (2, 3), ausgebildet sind.

2. Verfahren zur Herstellung von mehrphasigen Biomaterialien auf Basis bakteriell synthetisierter Nanocellulose (BNC), bei dem zumindest zwei unterschiedliche gemeinsam oder getrennt präparierte Cellulose-produzierende Bakterienstämme miteinander zu mehreren, unterschiedlichen Bakteriencellulose-Netzwerken in einem gemeinsamen Kulturmedium synthetisiert werden, wobei durch Auswahl der zumindest zwei unterschiedlichen Bakterienstämme, durch deren Präparation und Beimpfung sowie durch Beeinflussung der Synthesebedingungen die BNC-Struktur und die BNC-Eigenschaften der mehrphasigen Biomaterialien bestimmt werden, wobei die Bakteriencellulose-Netzwerke als kombiniertes homogenes Phasensystem (1) oder als schichtförmiges Phasensystem, bestehend aus wenigstens einer kombinierten homogenen Phase (1) sowie aus wenigstens einer Einzelphase (2, 3), synthetisiert werden.

3. Verfahren gemäß Anspruch 2, **dadurch gekennzeichnet, dass** die zumindest zwei unterschiedlichen Bakteriencellulose-Netzwerke unabhängig voneinander präpariet werden sowie anschließend zusammengeführt und gemeinsam synthetisiert werden.

4. Verfahren gemäß Anspruch 2, **dadurch gekennzeichnet, dass** die zumindest zwei unterschiedlichen Bakteriencellulose-Netzwerke für die gemeinsame Synthese bereits vor der Beimpfung zusammengeführt werden.

## Claims

1. Multiphase biomaterials on the basis of bacterially synthesized nanocellulose (BNC) consisting of at least two different bacterial cellulose networks, **characterized in that** the at least two different bacterial cellulose networks are designed as a combined homogenous phase system (1) or as a layered phase system consisting of at least one combined homogenous phase (1) as well as at least one single phase (2, 3).

2. Method for the production of multiphase biomaterials on the basis of bacterially synthesized nanocellulose (BNC) in which at least two different cellulose-producing bacterial strains prepared together or separated are synthesized together to several different bacterial cellulose networks in a common culture medium, wherein the BNC structure and the BNC properties of the multiphase biomaterials are affected by the choice of the at least two different bacterial strains, by their preparation and inoculation as well as by influencing the synthesis conditions, wherein the bacterial cellulose networks are synthesized as a combined homogenous phase system (1) or as a layered phase system consisting of at least one combined homogenous phase (1) as well as at least one single phase (2, 3).

3. Method according to claim 2, **characterized in that** the at least two different bacterial cellulose networks are prepared independently from each other and are subsequently brought together and are synthesized together.

4. Method according to claim 2, **characterized in that** for the conjoint synthesis the at least two different bacterial cellulose networks are brought together already before the inoculation.

## Revendications

1. Biomatériaux multiphasés à base de nanocellulose synthétisée par des bactéries (BNC), consistant en au moins deux réseaux de cellulose bactérienne différents, **caractérisés en ce que** les au moins deux réseaux de cellulose bactérienne différents sont conçus en tant que système de phases homogène combiné (1) ou en tant que système de phases formant une couche, consistant en au moins une phase homogène combinée (1) ainsi qu'en au moins une phase unique (2, 3).

2. Procédé de production de biomatériaux multiphasés à base de nanocellulose synthétisée par des bactéries (BNC), dans lequel au moins deux souches bactériennes différentes produisant de la cellulose préparées conjointement ou séparément sont synthétisées l'une avec l'autre en plusieurs réseaux de cellulose bactérienne différents dans un milieu de culture commun, dans lequel la structure de BNC et les propriétés de BNC des biomatériaux multiphasés sont déterminées par le choix des au moins deux souches bactériennes différentes, par leur préparation et par ensemencement ainsi que par l'influence des conditions de synthèse, dans lequel les réseaux de cellulose bactérienne sont synthétisés en tant que système de phases homogène combiné (1) ou en tant que système de phases formant une couche, consistant en au moins une phase homogène combinée (1) ainsi qu'au moins une phase unique (2, 3).

3. Procédé selon la revendication 2, **caractérisé en ce que** les au moins deux réseaux de cellulose bactérienne différents sont préparés indépendamment l'un de l'autre et ensuite sont joints et sont synthétisés conjointement.

4. Procédé selon la revendication 2, **caractérisé en ce que** les au moins deux réseaux de cellulose bactérienne différents sont joints pour la synthèse commune déjà avant l'ensemencement.
